(19) Europäisches Patentamt
European Patent Office
Office européen des brevets

(11) Publication number: **0 180 421 B1**

(12) **EUROPEAN PATENT SPECIFICATION**

(45) Date of publication of patent specification: **14.08.91**

(51) Int. Cl.⁵: **C07D 471/10**, C07D 498/10, C07D 513/10, A61K 31/435, //C07D215/20,(C07D471/10, 235:00,221:00)

(21) Application number: **85307712.1**

(22) Date of filing: **25.10.85**

The file contains technical information submitted after the application was filed and not included in this specification

(54) **Spiro-hydantoins for treatment of diabetic complications.**

(30) Priority: **30.10.84 PCT/US84/01767**

(43) Date of publication of application: **07.05.86 Bulletin 86/19**

(45) Publication of the grant of the patent: **14.08.91 Bulletin 91/33**

(84) Designated Contracting States: **AT BE CH DE FR GB IT LI LU NL SE**

(56) References cited: **FR-A- 2 367 754**

(73) Proprietor: **PFIZER INC.**
**235 East 42nd Street**
**New York, N.Y. 10017(US)**

(72) Inventor: **Lipinski, Christopher A.**
**10, Connshire Drive**
**Waterford, CT(US)**

(74) Representative: **Wood, David John et al**
**PFIZER LIMITED, Ramsgate Road**
**Sandwich, Kent CT13 9NJ(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 180 421 B1

## Description

This invention relates to novel spiro-hydantoins useful in the treatment of certain chronic complications arising from diabetes mellitus, such as diabetic cataracts, retinopathy and neuropathy, and to pharmaceutical compositions containing such compounds.

In the past, various attempts have been made to obtain more effective oral anti-diabetic agents. Generally, these efforts have involved synthesis of new organic compounds, particularly sulfonylureas, and determination of their ability to substantially lower blood sugar levels when administered orally. However, little is known about the effect of organic compounds in preventing or alleviating chronic complications or diabetes, such as diabetic cataracts, neuropathy and retinopathy. U.S. Patent No. 3,821,383 discloses aldose reductase inhibitors like 1,3-dioxo-1H-benz[d,e]-isoquinoline-2(3H)-acetic acid and derivatives thereof to be useful for the treatment of these conditions. U.S. Patent No. 4,117,230 teaches the use of certain hydantoins for treating complications of diabetes as aldose reductase inhibitors. Such aldose reductase inhibitors function by inhibiting the activity of the enzyme aldose reductase, which is primarily responsible for regulating the reduction of aldoses, such as glucose and galactose, to the corresponding polyols, such as sorbitol and galactitol, in humans and other animals. In this way, unwanted accumulations of galactitol in the lens of galactosemic subjects and of sorbitol in the lens, peripheral nervous cord and kidneys of various diabetic subjects are prevented or reduced. Accordingly, such compounds are of therapeutic value as aldose reductase inhibitors for controlling certain chronic diabetic complications, including those of an ocular nature, since it is known in the art that the presence of polyols in the lens of the eye leads to cataract formation, with a concomitant loss of lens clarity.

Carr et al., U.S Patent No. 3,985,888, teach certain spiroalkanone-imides and their use as sedatives. European Patent Application Publication No. 0065392 discloses certain spiro-succinimide derivatives and their use as aldose reductase inhibitors.

The compounds of the present invention are spiro-hydantoins of the formula;

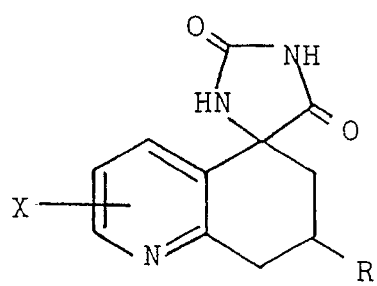

--- (I)

and their pharmaceutically acceptable salts wherein R is alkyl having 1-4 carbon atoms; and X is in the 3'-position and is hydrogen, halo, alkyl, alkoxy having 1-4 carbon atoms, alkylthio, alkylsulfinyl, alkylsulfonyl, phenoxy, phenylthio, or nitro; or X is in the 2'-position and is hydrogen, alkyl or alkoxy having 1-4 carbon atoms; wherein alkyl in each instance has 1-4 carbon atoms.

Preferably, R is methyl and X is H.

Both mixtures of optically active isomers and partially or completely optically resolved isomers of the compounds claimed herein are within the scope of the present invention.

Also embraced by the present invention are pharmaceutical compositions, comprising a pharmaceutically acceptable carrier or diluent and a compound of formula I.

The numbering system of the spiro-compounds of formula I is as shown.

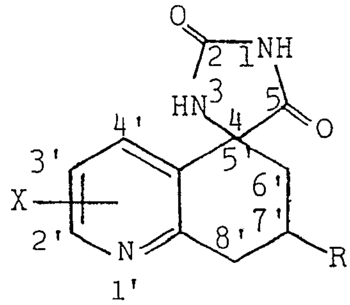

--- I

The compounds are derivatives of 7',8'-dihydro-spiro-[imidazolidine-4,5'(6'H)-quinoline]-2,5-diones and can be substituted by X in the 2'- or 3'-positions.

Diastereomers can be separated by methods known in the art, such as recrystallization with a suitable solvent like isopropanol, or trituration, for example, with an alcohol-ether solvent such as isopropanol-diethyl ether. The terms "Rel" and "(±)" each mean a 1:1 racemic mixture of the two optically active enantiomers.

When X is halo, halo includes fluoro, chloro, bromo and iodo.

In the Synthetic Scheme a preparation of compounds of formula I is shown. Starting diketone II wherein R is alkyl having 1-4 carbon atoms is reacted with ammonia in a refluxing solvent, such as benzene, which will remove the water of reaction as an azeotrope to obtain ketone eneamine III. When X is hydrogen, III is reacted with propynal in a polar aprotic organic solvent such as dimethylformamide at an initial temperature of between -10° and 25° C, preferably about 0° C, followed by a period of heating of between 15 and 90 minutes, preferably about 45 minutes, at a temperature of between 100° and 153° C, preferably about 153° C, to obtain tetrahydroquinoline derivative IV where X is hydrogen.

When X is other than hydrogen, alkene aldehyde, V or VI, which are known compounds or can be prepared analogously to the known compounds, is reacted with ketone eneamine III in a polar, aprotic organic solvent such as dimethylformamide at between -10° and 25° C, followed by heating at between 100° and 153° C, to obtain tetrahydroquinoline derivative IV.

SYNTHETIC SCHEME

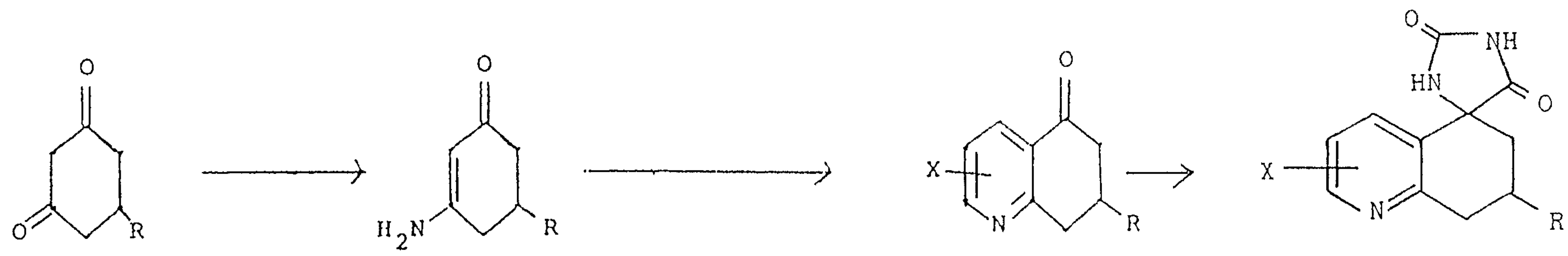

II III IV I

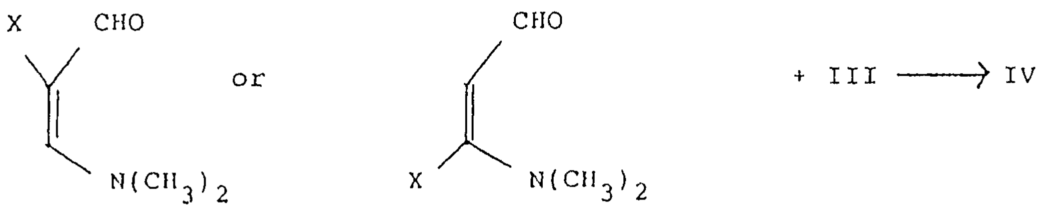

V or VI + III → IV

SYNTHETIC SCHEME (Continued)

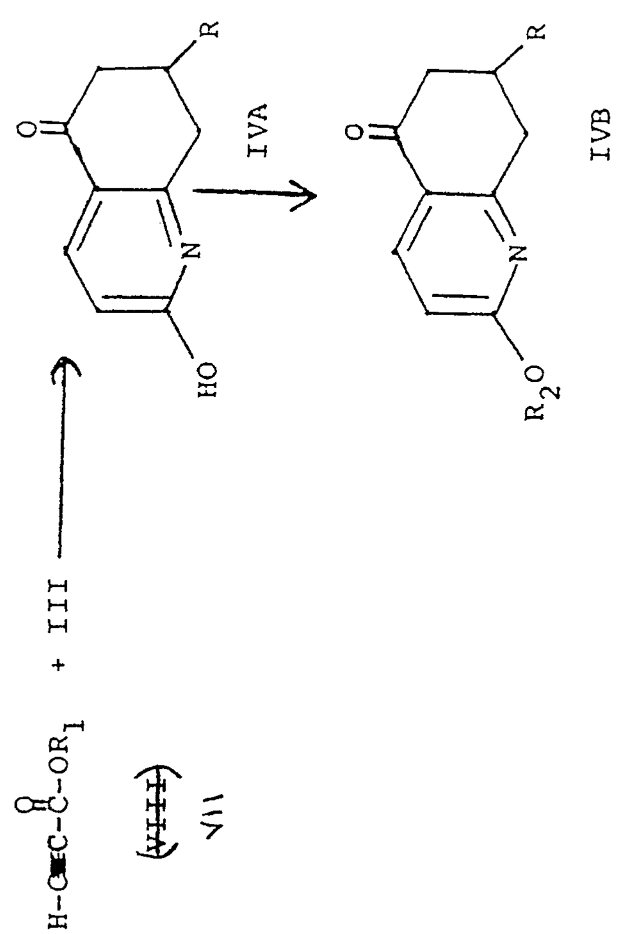

H-C≡C-C(=O)-OR1 (VII) + III → IVA → IVB

The compound of formula IV can be further reacted to obtain the corresponding compounds of formula I, for example, by means of the methods described in U.S. Patent No. 4,117,230. A compound of formula IV is condensed with an alkali metal cyanide (e.g., sodium cyanide or potassium cyanide) and ammonium carbonate to form the desired spiro-hydantoin final product. This reaction is normally carried out in the presence of an aqueous reaction-inert polar organic solvent medium in which both the reactants and reagents are mutually miscible. Preferred organic solvents include cyclic ethers such as dioxane and tetrahydrofuran, lower alkylene glycols like ethylene glycol and trimethylene glycol, water-miscible lower alkanols such as methanol, ethanol and isopropanol, as well as N,N-di(lower alkyl) lower alkancamides like N,N-dimethylformamide, N,N-diethylformamide and N,N-dimethylacetamide, etc. In general, the reaction is conducted at a temperature that is in the range of from 20° C. up to 120° C. for a period of two hours to four days. Although the amount of reactant and reagents employed in the reaction can vary to some extent, it is preferable to employ at least a slight molar excess of the alkali metal cyanide reagent with respect to the carbonyl ring compound starting material in order to effect maximum yield. Upon completion of the reaction, the desired product is easily isolated in a conventional manner, e.g., by first diluting the reaction mixture with water (boiling if necessary) and then cooling the resultant aqueous solution to room temperature, followed by acidification to afford the spiro-hydantoin compound in the form of a readily-recoverable precipitate.

When X is 2'-alkoxy, the preferred method of preparing the corresponding compounds of formula I is according to the procedure of Dubas-Sluyter et al., Recueil Chim. Pays Bas, 91, 157-160 (1972) wherein a compound of formula III is reacted with an alkyl propiolate VII wherein $R_1$ is alkyl having 1-4 carbon atoms, preferably methyl, in a polar aprotic solvent such as dimethylformamide or carbitol at a temperature range of between 100° and 153° C., preferably about 153° C., to obtain the hydroxy compound of formula IVA. It

is to be understood that the compound of the formula IVA may also be present as the 2-pyridone isomer.

The compound of formula IVA is reacted with a water soluble inorganic silver salt such as silver nitrate and aqueous base such as potassium hydroxide at a pH of between 9 and 12, preferably about 10.5, and at a temperature of between 0° and 60° C, preferably about 25° C, to form a silver salt. The isolated silver salt is reacted with an alkyl iodide of the formula $R_2$-I wherein $R_2$ is alkyl having 1-4 carbon atoms at a temperature of between 25° and 100° C, preferably 60° C, to obtain the 2-alkoxy quinoline derivative IVB. The conversion to compounds of formula I continues according to the procedures set forth for a compound of formula IV.

Because of the acidic hydrogen atom in the spiro-5-membered heterocyclic ring of the compounds of formula I, salts may be formed with pharmaceutically acceptable cations by conventional methods. Thus, these salts may be readily prepared by treating the compound of formula I with an aqueous solution of the desired pharmaceutically acceptable cation and evaporating the resulting solution to dryness, preferably under reduced pressure. Alternatively, a lower alkyl alcohol solution of the compound of formula I may be mixed with an alkoxide of the desired cation and the resulting solution subsequently evaporated to dryness. Suitable pharmaceutically acceptable cations for this purpose include, but are not limited to, alkali metal catons such as potassium and sodium, alkaline earth metal cations such as calcium and magnesium, ammonium, lower alkanol-ammonium and other cations derived from pharmaceutically acceptable organic amines which can form water-soluble amine addition salts.

Acid addition salts can be formed for compounds of formula I Suitable salts include those derived from hydrochloric acid, sulfuric acid or methylsulfonic acid. These acid addition salts can be prepared by the addition of the appropriate strong acid to a lower alcoholic solution of a compound of formula I at a temperature of 0°-60° C., preferably 25° C., followed by concentration to obtain the desired product. Alternatively, an aqueous slurry of a compound of formula I can be mixed with the appropriate strong acid at 0°-60° C., preferably about 25° C., followed by freeze drying and recrystallization from a lower alcohol.

Pharmaceutically acceptable salts are those which do not cause unacceptable adverse reactions when administered.

The novel compounds of formula I and the pharmaceutically acceptable salts thereof are useful as inhibitors of the enzyme aldose reductase in the treatment of chronic complications of diabetes, such as diabetic cataracts, retinopathy and neuropathy. As used in the claims and specification hereof, treatment is meant to include both the prevention and alleviation of such conditions. The compound may be administered to a subject in need of treatment by a variety of conventional routes of administration, including orally, parenterally and topically. In general, these compounds will be administered orally or parenterally at dosages between 0.05 and 25 mg./kg. body weight of the subject to be treated per day, preferably from 0.1 to 10 mg./kg. per day. However, some variation in dosage will necessarily occur depending on the condition of the subject being treated. The person responsible for administration will, in any event, determine the appropriate dose for the individual subject.

The novel compounds of the invention may be administered alone or in combination with pharmaceutically acceptable carriers, in either single or multiple doses. Suitable pharmaceutical carriers include inert solid diluents or fillers, sterile aqueous solutions and various organic solvents. The pharmaceutical compositions formed by combining the novel compounds of formula I and the pharmaceutically acceptable carriers are then readily administered in a variety of dosage forms such as tablets, powders, lozenges, syrups or injectable solutions. These pharmaceutical compositions can, if desired, contain additional ingredients such as flavorings, binders or excipients. Thus, for purposes of oral administration, tablets containing various excipients such as sodium citrate, calcium carbonate and calcium phosphate may be employed along with various disintegrants such as starch, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid compositions of a similar type may also be employed as fillers in soft and hard filled gelatin capsules. Preferred materials for this include lactose or milk sugar and high molecular weight polyethylene glycols. When aqueous suspensions or elixirs are desired for oral administration, the essential active ingredient therein may be combined with various sweetening or flavoring agents, coloring matter or dyes and, if desired, emulsifying or suspending agents, together with diluents such as water, ethanol, propylene glycol, glycerin and combinations thereof.

For parenteral administration, solutions of the novel compound of formula I in sesame or peanut oil, aqueous propylene glycol, or in sterile aqueous solution may be employed. Such aqueous solutions should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, the sterile aqueous media employed are all

readily available by standard techniques known to those skilled in the art.

Compounds of formula I may not only be advantageously employed for the preparation of aqueous pharmaceutical compositions for parenteral administration, as described above, but more particularly for the preparation of pharmaceutical compositions suitable for use as ophthalmic solutions. Such ophthalmic solutions are of principal interest for the treatment of diabetic cataracts by topical administration and the treatment of such conditions in this manner is a preferred embodiment of the present invention. Thus, for the treatment of diabetic cataracts the compounds of this invention are administered to the eye of the subject in need of treatment in the form of an ophthalmic preparation prepared in accordance with conventional pharmaceutical practice, see for example "Remington's Pharmaceutical Sciences", 15th Edition, pages 1488 to 1501 (Mack Publishing Co., Easton, Pa.). The ophthalmic preparation will contain a compound of formula I, or a pharmaceutically acceptable salt thereof, in a concentration from 0.01 to 1% by weight, preferably from 0.05 to 0.5%, in a pharmaceutically acceptable solution, suspension or ointment. Some variation in concentration will necessarily occur, depending on the particular compound employed and the condition of the subject to be treated, and the person responsible for treatment will determine the most suitable concentration for the individual subject. The ophthalmic preparation will preferably be in the form of a sterile aqueous solution containing, if desired, additional ingredients, for example preservatives, buffers, tonicity agents, antioxidants and stabilizers, nonionic wetting or clarifying agents or viscosity-increasing agents. Suitable preservatives include benzalkonium chloride, benzethonium chloride, chlorobutanol or thimerosal. Suitable buffers include boric acid, sodium and potassium bicarbonate, sodium and potasssium borate, sodium and potassium carbonate, sodium acetate sodium biphosphate, in amounts sufficient to maintain the pH at between 6 and 8, preferably between 7 and 7.5. Suitable tonicity agents are Dextran 40, Dextran 70 (Trade Marks), dextrose, glycerin, potassium chloride, propylene glycol, sodium chloride, and the like, such that the sodium chloride equivalent of the ophthalmic solution is in the range 0.9 plus or minus 0.2%. Suitable antioxidants and stabilizers include sodium bisulfite, sodium metabisulfite, sodium thiosulfite or thiourea. Suitable wetting and clarifying agents include Polysorbate 80, Polysorbate 20, Poloxamer 282 and Tyloxapol (Trade Marks). Suitable viscosity-increasing agents include Dextran 40, Dextran 70(Trade Marks), gelatin, glycerin, hydroxyethylcellulose, hydroxmethylpropylcellulose, lanolin, methylcellulose, petrolatum, polyethylene glycol, polyvinyl alcohol, polyvinylpyrrolidone or carboxymethylcellulose.

The ophthalmic preparation will be administered topically to the eye of the subject in need of treatment by conventional methods, for example in the form of drops or by bathing the eye in the ophthalmic solution.

The activity of the compounds of the present invention as agents for the control of chronic diabetic complications may be determined by a number of standard biological or pharmacological tests. Suitable tests include (1) measuring their ability to inhibit the enzyme activity of isolated aldose reductase; (2) measuring their ability to reduce or inhibit sorbitol accumulation in the sciatic nerve and lens of acutely streptozotocinized, i.e. diabetic, rats; (3) measuring their ability to reverse already-elevated sorbitol levels in the sciatic nerve and lens of chronic streptozotocin-induced diabetic rats; (4) measuring their ability to prevent or inhibit galactitol formation in the lens of acutely galactosemic rats; (5) measuring their ability to delay cataract formation and reduce the severity of lens opacities in chronic galactosemic rats; (6) measuring their ability to prevent sorbitol accumulation and cataract formation in isolated rat lens incubated with glucose; and (7) measuring their ability to reduce already elevated sorbitol levels in isolated rat lens incubated with glucose.

The present invention is illustrated by the following examples. It will be understood, however, that the invention is not limited to the specific details of these examples. Proton nuclear magnetic resonance spectra (NMR) were measured at 250 MHz (unless otherwise indicated) for solutions in perdeuterodimethyl sulfoxide (DMSO-$d_6$) and peak positions are expressed in parts per million (ppm) downfield from tetramethylsilane. The peak shapes are denoted as follows: s, singlet; d, doublet; t, triplet; q, quartet; m, multiplet; b, broad; very, v.

EXAMPLE 1

rel 4,5'S 7'S Spiro[imidazolidine-4,5'(6'H)-quinoline]-2,5-dione-7',8'-dihydro-7'-methyl

To a solution of 13.3 g (0.138 mol) of ammonium carbonate and 3.8 g (0.058 mol) of potassium cyanide in 53 ml of water was added a solution of 4.75 g (0.029 mol) of 7-methyl-7,8-dihydro-quinolin-5(6H)-one in 53 ml of ethanol. The reaction was heated at 65°C for 72 hours. The solution was cooled and filtered and the filtrate was brought to pH 6.5 at which point a gummy solid precipitated. The solid was filtered and washed well with water. Recrystallization from ethanol gave 2.0 g of the title compound: mp. 252-254°C as

a mixture composed of 80% of the rel 7'S methyl diastereomer and 20% of the rel 7'R methyl diastereomer. NMR (DMSO-$d_6$): δ 11.03 (vbs, 1H); 8.76 (s, 0.8 x 1H); 8.47 (m, 1H); 8.36 (s, 0.2 x 1H); 7.68 (m, 0.2 x 1H); 7.46 (m, 0.8 x1H); 7.27 (m, 1H); 2.85-3.0 (m, 1H); 2.35-2.6 (m, 1H); 2.0-2.2 (m, 1H); 1.75-2.0 (m, 0.8x1H); 1.64 (t, 0.2x1H); 1.09 (d, 0.8x3H); and 1.05 (d, 0.2x3H) ppm.

EXAMPLE 2

rel 4,5'S 7'R Spiro[imidazolidine-4,5'(6'H)-quinoline]-2,5-dione-7',8'-dihydro-7'-methyl

Concentration of the ethanol mother liquors from preparation of material enriched in the rel 4,5'S 7'R diastereomer of Example 1 and recrystallization from ethyl acetate gave 1.45 g of material which was recrystallized from water to give 860 mg of rel 4,5'S 7'R spiro[imidazolidine-4,5'(6'H)-quinoline]-2,5-dione-7',8'-dihydro-7'-methyl: mp 145-152°C as a mixture of 90% rel 7'R methyl diastereomer and 10% rel 7'S methyl diastereomer. NMR (DMSO-$d_6$): δ 10.88 (vb s, 1, NH); 8.76 (s, 0.1x1, NH); 8.5 (m,1H); 8.36 (s, 0.9x1,NH); 7.68 (m, 0.9x1H); 7.46 (m, 0.1x1H); 7.27 (m, 1H); 2.9-3.05 (m,1H); 2.5-2.75 (m, 1H); 2.35-2.5 (m,1H); 2.05-2.15 (bd, 1H); 1.75-1.95 (m, 0.1 x1H); 1.64 (t, 0.9x1H), 1.09 (d, 0.1x3H); and 1.05 (d, 0.9x3H) ppm.

EXAMPLE 3

Spiro[imidazoline-4,5'(6'H)-quinoline]-2,5-dione 3'-chloro-7',8'-dihydro-7'-methyl

To a solution of 28.8 mg (0.44 mmol) of potassium cyanide and 100 mg (1.04 mmol) of ammonium carbonate in 4 ml of ethanol and 4 ml of water was added 40 mg (0.204 mmol) of 3-chloro-7-methyl-7,8-dihydroquinolin-5(6H)-one and the reaction was stirred at 65°C for 24 hours. An additional 101 mg of ammonium carbonate was added and heating was continued for 72 hours. An additional 101 mg of ammonium carbonate and 3 ml ethanol and 2 ml water, was added and heating was continued at 65°C for 24 hours. An additional 101 mg of ammonium carbonate was added and heating was continued for 6 days. The ethanol was removed from the dark reaction solution by concentration in vacuo. The residue was diluted with water and the pH was brought to 7 with aqueous hydrochloric acid and the reaction was extracted with two 50 ml portions ethyl acetate. After drying over anhydrous sodium sulfate, the solution was concentrated in vacuo to 30 mg of a dark green solid. High resolution mass spectroscopic analysis confirmed the presence of the title compound. Calculated for $C_{12}H_{12}N_3O_2Cl$ exact mass,m/e 265.0619. Found: 265.0624.

PREPARATION

3-Chloro-7-methyl-7,8-dihydroquinolin-5(6H)-one

To 2.14 g (0.018 mol) of 2-chloro-3-dimethylaminoacrolein in 20 ml dimethylformamide was added 2.25 g (0.018 mol) of 3-amino-5-methyl-cyclohex-2-enone. The reaction was heated at reflux for 40 hours. Most of the solvent was removed in vacuo and the residue was triturated with petroleum ether and concentrated in vacuo. This procedure was repeated three times. The resultant amber oil was triturated with 50 ml of ethyl acetate and filtered to remove an insoluble black powder. The ethyl acetate mother liquors were concentrated in vacuo to give 1.54 g of a dark brown oil. This material (0.93 g) was subjected to column chromatography on silica gel using ethyl acetate as eluent. Material with $R_f$ of 0.45 was isolated as a brown oil. The material was identified as largely containing the title compound based on the following spectral data. Mass spectrum base peak, m/e 195, with ratio 195:197 of 3:1 corresponding to chlorine isotopes. NMR (DMSO-$d_6$): δ 8.78 (d, 1H, J = 2.5Hz); 8.15 (d, 1H, J = 2.5Hz); 2.2-3.2 (m, 5H); and 1.14 (d, 5H) ppm.

**Claims**

**Claims for the following Contracting States : BE, CH, DE, FR, GB, IT, LI, LU, NL, SE**

**1.** A spiro-hydantoin compound of the formula:

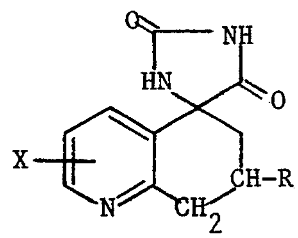

or a pharmaceutically acceptable salt thereof, wherein

R is alkyl having 1-4 carbon atoms;

X is in the 3'-position and is hydrogen, halo, alkyl, alkoxy having 1-4 carbon atoms, alkylthio, alkylsulfinyl, alkylsulfonyl, phenoxy, phenylthio, or nitro; or X is in the 2'-position and is hydrogen, alkyl, or alkoxy having 1-4 carbon atoms; wherein alkyl in each instance has 1-4 carbon atoms.

2. A compound according to claim 1 wherein R is methyl and X is hydrogen.

3. A pharmaceutical composition comprising a compound according to claim 1 or claim 2 and a pharmaceutically acceptable salt thereof for use in medicine.

4. A compound as claimed in claim 1 or 2, or a pharmaceutically acceptable salt thereof, for use as a medicament.

5. The use of a compound as claimed in claim 1 or 2, or of a pharmaceutically acceptable salt thereof, for the manufacture of a medicament for use in the treatment of diabetic cataracts, retinopathy or neuropathy.

**Claims for the following Contracting State : AT**

1. A process for preparing a spiro-hydantoin compound of the formula:

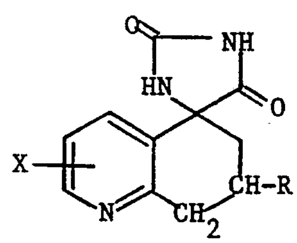

--- (I)

or a pharmaceutically acceptable salt thereof, wherein R is 1-4 carbon atoms; and X is in the 3'-position and is hydrogen, halo, alkyl, alkoxy having 1-4 carbon atoms, alkylthio, alkylsulfinyl, alkylsulfonyl, phenoxy, phenylthio, or nitro; or X is in the 2'-position and is hydrogen, alkyl, or alkoxy having 1-4 carbon atoms; wherein alkyl in each instance has 1-4 carbon atoms; characterized by (a) condensing an appropriately substituted carbonyl ring compound of the formula:

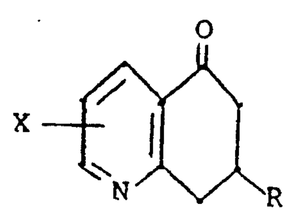

IV

wherein X is as previously defined and R is alkyl having 1-4 carbon atoms, with an alkali metal cyanide and ammonium carbonate to form the corresponding spiro-hydantoin final product of the formula:

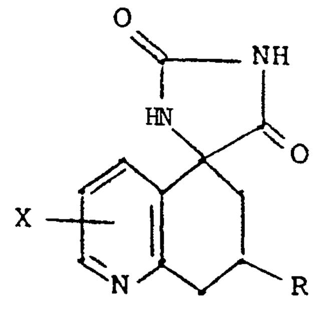

IA

and thereafter, if desired, converting a compound of formula IA to a pharmaceutically acceptable salt thereof, whereby a spiro-hydantoin compound of formula I or a pharmaceutically salt thereof is obtained.

2. A process as claimed in part (a) of claim 1, characterized by the fact that said condensation reaction is carried out by employing a slight molar excess of the alkali metal cyanide reagent with respect to the carbonyl ring compound starting material.

3. A process as claimed in part (a) of claim 1, characterized by the fact that said condensation reaction is carried out in an aqueous reaction-inert polar organic solvent medium at a temperature that is in the range of from about 20° C. to about 120° C.

4. A process as claimed in claim 3, characterized by the fact that said organic solvent is a water-miscible lower alkanol.

5. The process as claimed in part (a) of claim 1 wherein the spiro-hydantoin compound prepared is rel 4,5'S 7'R spiro[imidazolidine-4,5' (6'H)-quinoline]-2,5-dione-7',8'-dihydro-7'-methyl.

6. A process for preparing a pharmaceutical composition characterized by mixing a compound of the formula (I) as defined in claim 1 or a pharmaceutically acceptable salt thereof, with a pharmaceutically acceptable diluent or carrier.

**Revendications**

**Revendications pour les Etats contractants suivants : BE, CH, DE, FR, GB, IT, LI, LU, NL, SE**

1. Composé spiro-hidantoïne de formule :

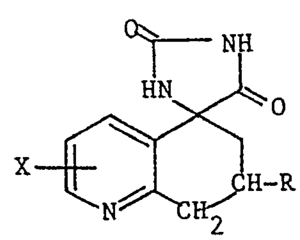

ou sel pharmaceutiquement acceptable d'un tel composé, dans laquelle

R est un groupe alkyle ayant de 1 à 4 atomes de carbone ;

X est en position-3' et représente l'hydrogène, un groupe halogéno, alkyle, alcoxy ayant de 1 à 4 atomes de carbone, alkylthio, alkylsulfinyle, alkylsulfonyle, phénoxy, phénylthio, ou nitro ; ou X est en position-2' et représente l'hydrogène, un groupe alkyle ou alcoxy ayant de 1 à 4 atomes de carbone ;

le groupe alkyle ayant de 1 à 4 atomes de carbone dans chaque cas.

2. Composé selon la revendication 1, dans lequel R est un groupe méthyle et X représente l'hydrogène.

3. Composition pharmaceutique comprenant un composé selon la revendication 1 ou 2 et un sel pharmaceutiquement acceptable d'un tel composé en vue de son utilisation en médecine.

4. Composé selon la revendication 1 ou 2, ou sel pharmaceutiquement acceptable d'un tel composé, destiné à son usage comme médicament.

5. Utilisation d'un composé selon la revendication 1 ou 2, ou d'un sel pharmaceutiquement acceptable d'un tel composé, pour la fabrication d'un médicament destiné au traitement des cataractes, rétinopathies et neuropathies diabétiques.

**Revendications pour l'Etat contractant suivant : AT**

1. Procédé de préparation d'un composé spiro-hidantoïne de formule :

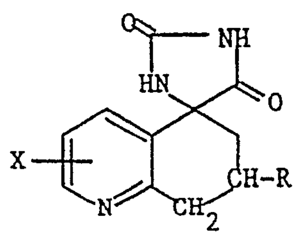

---(I)

ou d'un sel pharmaceutiquement acceptable d'un tel composé, dans laquelle

R est un groupe alkyle ayant de 1 à 4 atomes de carbone ; et X est en position-3' et représente l'hydrogène, un groupe halogéno, alkyle, alcoxy ayant de 1 à 4 atomes de carbone, alkylthio, alkylsulfinyle, alkylsulfonyle, phénoxy, phénylthio, ou nitro ; ou X est en position-2' et représente l'hydrogène, un groupe alkyle ou alcoxy ayant de 1 à 4 atomes de carbone ; le groupe alkyle ayant de 1 à 4 atomes de carbone dans chaque cas ; caractérisé en ce qu'il consiste (a) à condenser un composé cyclique carbonyle convenablement substitué de formule :

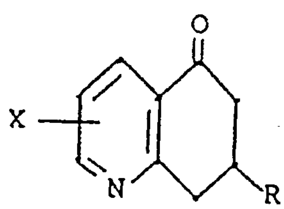

IV

dans laquelle X est tel que précédemment défini et R est un groupe alkyle ayant de 1 à 4 atomes de carbone, avec un cyanure de métal alcalin et du carbonate d'ammonium pour former le produit spiro-hidantoïne final de formule :

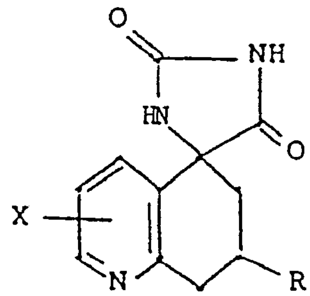

IA

puis, si on le désire, à transformer un composé de formule (IA) en un sel pharmaceutiquement acceptable d'un tel composé, grâce à quoi l'on obtient un composé spiro-hidantoïne de formule (I) ou un sel pharmaceutiquement acceptable d'un tel composé.

2. Procédé tel que revendiqué dans la partie (a) de la revendication 1, caractérisé par le fait que ladite réaction de condensation est mise en oeuvre en utilisant un léger excès molaire de réactif cyanure de métal alcalin par rapport au composé cyclique carbonyle servant de matière de départ.

3. Procédé tel que revendiqué dans la partie (a) de la revendication 1, caractérisé par le fait que ladite réaction de condensation est mise en oeuvre dans un milieu solvant organique polaire inerte vis-à-vis du milieu de réaction aqueux à une température comprise dans la gamme allant d'environ 20°C à environ 120°C.

4. Procédé selon la revendication 3, caractérisé par le fait que ledit solvant organique est un alcanol inférieur miscible à l'eau.

5. Procédé tel que revendiqué dans la partie (a) de la revendication 1, dans lequel le composé spiro-hidantoïne préparé est le Rel 4,5'S 7'R 7',8'-dihydro-7'-méthyl-spiro-(imidazolidine-4,5' (6'H)quinoline)-2,5-dione.

6. Procédé de préparation d'une composition pharmaceutique caractérisé en ce qu'il consiste à mélanger un composé de formule (I) tel que défini dans la revendication 1, ou un sel pharmaceutiquement acceptable d'un tel composé avec un diluant ou véhicule pharmaceutiquement acceptable.

**Patentansprüche**

**Patentansprüche für folgende Vertragsstaaten : BE, CH, DE, FR, GB, IT, LI, LU, NL, SE**

1. Spirohydantoinverbindung der Formel

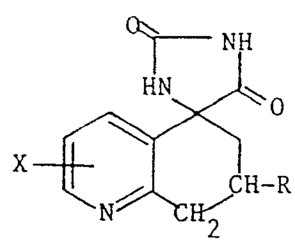

oder ein pharmazeutisch annehmbares Salz hievon, worin R Alkyl mit 1 bis 4 Kohlenstoffatomen darstellt; X in Stellung 3' ist und Wasserstoff, Halogen, Alkyl, Alkoxy mit 1 bis 4 Kohlenstoffatomen, Alkylthio, Alkylsulfinyl, Alkylsulfonyl, Phenoxy, Phenylthio oder Nitro bedeutet; oder X in Stellung 2' ist und Wasserstoff, Alkyl oder Alkoxy mit 1 bis 4 Kohlenstoffatomen darstellt; wobei Alkyl in jedem Fall 1 bis 4 Kohlenstoffatomen aufweist.

2. Verbindung gemäß Anspruch 1, worin R Methyl und X Wasserstoff bedeutet.

3. Pharmazeutische Zusammensetzung, umfassend eine Verbindung gemäß Anspruch 1 oder 2 oder ein pharmazeutisch annehmbares Salz hievon zur medizinischen Verwendung.

4. Verbindung gemäß Anspruch 1 oder 2 oder ein pharmazeutisch annehmbares Salz hievon zur Verwendung als Medikament.

5. Verwendung einer Verbindung gemäß Anspruch 1 oder 2 oder eines pharmazeutisch annehmbaren Salzes hievon zur Herstellung eines Medikaments zur Verwendung bei der Behandlung von diabetischen Katarakten, Retinopathie und Neuropathie.

**Patentansprüche für folgenden Vertragsstaat : AT**

1. Verfahren zur Herstellung einer Spirohydantoinverbindung der Formel (I)

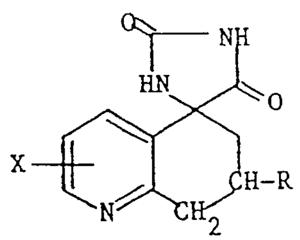

(I)

oder eines pharmazeutisch annehmbaren Salzes hievon, worin R Alkyl mit 1 bis 4 Kohlenstoffatomen darstellt; X in Stellung 3' ist und Wasserstoff, Halogen, Alkyl, Alkoxy mit 1 bis 4 Kohlenstoffatomen, Alkylthio, Alkylsulfinyl, Alkylsulfonyl, Phenoxy, Phenylthio oder Nitro bedeutet; oder X in Stellung 2' ist und Wasserstoff, Alkyl oder Alkoxy mit 1 bis 4 Kohlenstoffatomen darstellt; wobei Alkyl in jedem Fall 1 bis 4 Kohlenstoffatomen aufweist, dadurch gekennzeichnet, daß (a) eine geeignet substituierte Carbonylringverbindung der Formel (IV)

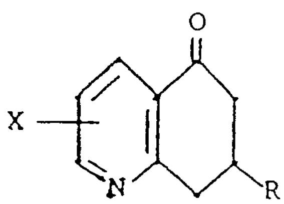

(IV),

worin X wie oben definiert ist und R Alkyl mit 1 bis 4 Kohlenstoffatomen darstellt, mit einem Alkalimetallcyanid und Ammoniumcarbonat kondensiert wird, wobei das entsprechende Spirohydantoinendprodukt der Formel (IA)

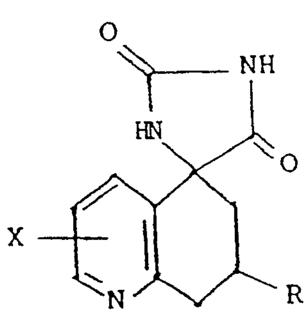

(IA)

gebildet wird, und danach gewünschtenfalls eine Verbindung der Formel (IA) in ein pharmazeutisch annehmbares Salz hievon übergeführt wird, wobei eine Spirohydantoinverbindung der Formel (I) oder ein pharmazeutisch annehmbares Salz hievon erhalten wird.

2. Verfahren gemäß Teil (a) von Anspruch 1, dadurch gekennzeichnet, daß die Kondensationsreaktion unter Verwendung eines geringen molaren Überschußes des Alkalimetallcyanidreagens in Bezug auf das Carbonylringverbindungs-Ausgangsmaterial durchgeführt wird.

3. Verfahren gemäß Teil (a) von Anspruch 1, dadurch gekennzeichnet, daß die Kondensationsreaktion in einem wässerigen reaktionsinerten polaren organischen Lösungsmittelmedium bei einer Temperatur im Bereich von etwa 20° C bis etwa 120° C durchgeführt wird.

4. Verfahren gemäß Anspruch 3, dadurch gekennzeichnet, daß das organische Lösungsmittel ein mit Wasser mischbares niederes Alkanol ist.

5. Verfahren gemäß Teil (a) von Anspruch 1, worin die hergestellte spiro-Hydantoinverbindung rel-4,5'S,7'R-spiro-[Imidazolidin-4,5'(6'H)-chinolin]-2,5-dion-7',8'-dihydro-7'-methyl ist.

6. Verfahren zur Herstellung einer pharmazeutischen Zusammensetzung, dadurch gekennzeichnet, daß eine Verbindung der Formel (I), wie in Anspruch 1 definiert, oder ein pharmazeutisch annehmbares Salz hievon mit einem pharmazeutisch annehmbaren Verdünnungsmittel oder Träger gemischt wird.